# EUROPEAN PATENT SPECIFICATION

(11) **EP 1 210 753 B1**
(45) Date of publication and mention of the grant of the patent: **15.02.2006**
(21) Application number: 00986181.6
(22) Date of filing: 18.08.2000
(51) Int. Cl.: H01S 5/00

(54) **TUNABLE LASER SOURCE WITH INTEGRATED OPTICAL AMPLIFIER**
ABSTIMMBARE LASERQUELLE MIT INTEGRIERTEM OPTISCHEN VERSTÄRKER
SOURCE LASER ACCORDABLE, A AMPLIFICATEUR OPTIQUE INTEGRE

(30) Priority: 02.09.1999 US 152049 P; 02.09.1999 US 152038 P; 02.09.1999 US 152072 P; 12.07.2000 US 614377; 12.07.2000 US 614895; 12.07.2000 US 614674; 12.07.2000 US 614378; 12.07.2000 US 614376; 12.07.2000 US 614195; 12.07.2000 US 614375; 12.07.2000 US 614865; 12.07.2000 US 614224
(43) Date of publication of application: 05.06.2002
(73) Proprietor: Agility Communications, Inc., Goleta, CA 93117 (US)
(72) Inventor: MASON, Thomas, Beck, Bethlehem, Pennsylvania 18015 (US); FISH, Gregory, Santa Barbara, CA 93110 (US); COLDREN, Larry, Santa Barbara, CA 93110 (US)
(74) Representative: Haines, Miles John L.S.
(86) International application number: PCT/US2000/022816
(87) International publication number: WO 2001/017076

(56) References cited:
- EP-A- 0 620 475
- GB-A- 1 539 028
- US-A- 5 088 105
- US-A- 5 715 268
- LEE S -L ET AL: "SAMPLED GRATING DBR LASER ARRAYS WITH ADJUSTABLE 0.8/1.6-NM WAVELENGTH SPACING" IEEE PHOTONICS TECHNOLOGY LETTERS,IEEE INC. NEW YORK,US, vol. 11, no. 8, August 1999 (1999-08), pages 955-957, XP000860962 ISSN: 1041-1135
- VIJAYSEKHAR JAYARAMAN ET AL: "THEORY, DESIGN, AND PERFORMANCE OF EXTENDED TUNING RANGE SEMICONDUCTOR LASERS WITH SAMPLED GRATINGS" IEEE JOURNAL OF QUANTUM ELECTRONICS,IEEE INC. NEW YORK,US, vol. 29, no. 6, 1 June 1993 (1993-06-01), pages 1824-1834, XP000397620 ISSN: 0018-9197

## Description

### BACKGROUND OF THE INVENTION

### Field of the Invention

This invention relates generally to laser assemblies, and more particularly to a widely tunable laser assembly with an integrated optical amplifier.

### Brief description of the Related Art

Thin fibers of optical materials transmit light across a very broad frequency bandwidth and therefore communications data from a light source may be transmitted over such fibers over broad frequency ranges. At any particular frequency, a laser source must have high output power, narrow laser linewidth and good transmission performance through great distances of optical fiber.

U.S. Patent No. 5,715,268, issued February 3, 1998 to Lang, describes a typical laser source. In Lang, a travelling-wave semiconductor laser amplifier having suppressed self-oscillation is provided. When incorporated into a master oscillator power amplifier device, such a device has improved light output versus amplifier current characteristics. Also provided is a method for suppressing self-oscillation in travelling-wave semiconductor laser amplifier structures for improving the characteristics of the device into which the amplifier is incorporated.

Alternative laser amplifiers are described in U.S. Patent No. 5,088,105, issued February 11, 1992 to Scifres et al. An optical amplifier has one or more amplifier regions with a noncollinear light path provided by curved or folded waveguides therein between input, output and reflective surfaces provided, for example, by a low reflectivity front facet and a high reflectivity rear facet. The amplifier regions are electrically pumped via conductive contacts which may be individually addressable for each amplifier region to provide phase control of the array of emitted light. Light is accepted through the front facet by a first amplifier region, is reflected from the rear facet and is emitted through the front facet. If there are multiple amplifier regions, a portion of the light is reflected by the front facet into an adjacent amplifier region. The light path is incident on the front and rear facets at an angle other than normal thereto and preferably at most 10 degrees from normal, and is confined to an optically active region. The light source may be a laser diode which can be monolithically integrated on the same substrate as the amplifier to form a master oscillator power amplifier (MOPA) device. The laser can be wavelength tunable. The MOPA can also have a steerable output beam.

In higher bandwidth communications systems, where many frequencies of laser light are transmitted along a fiber, there may be one or several laser sources. While a tunable laser source would be preferred, higher data capacity systems presently use multiple laser sources operating on different frequency channels to cover the wide fiber transmission bandwidth. This is the case since appropriate laser sources are presently incapable of rapid, electronic frequency tuning without attendant deterioration of other significant figures-of-merit.

For example, at a fixed frequency, sampled grating distributed Bragg reflector (SGDBR) lasers have the high output power, narrow laser linewidth and good transmission performance necessary for an optical data network. While some SGDBR lasers can be rapidly tuned over more than 100 different transmission channels, two problems nevertheless prevent these devices from being employed in fiber optic communication systems. The most significant problem is the significant absorption of the mirror material. The resulting large cavity losses act to make the laser output power insufficient for the requirements of a present-day communications system. A second problem is that the output power and frequency tuning are dependent on each other. This coupling results in inadequate controllability for a present-day communications system.

What is needed, instead, is a device with a combination of sufficiently high output power for a high-bandwidth optical communications network and with frequency tuning controllability substantially independent of output power controllability.

### SUMMARY

Accordingly, an object of the present invention is to provide an integrated laser assembly that includes a tunable solid state laser and optical amplifier where all of the elements are fabricated in a common epitaxial layer structure.

Another object of the present invention is to provide an integrated laser assembly that includes a tunable solid state laser and optical amplifier with an output mode conditioned for transmission in an optical fiber.

Another object of the present invention is to provide an integrated laser assembly that includes a tunable laser and optical amplifier reducing optical feedback from the amplifier to the laser.

A further object of the present invention is to provide a tunable, integrated laser assembly where laser frequency control and output power control are substantially independent.

These and other objects of the present invention are achieved in a laser assembly that includes an epitaxial structure formed on a substrate. A tunable laser resonator and a separately controllable optical amplifier are formed in the common epitaxial structure. The amplifier is positioned outside of the laser resonator cavity to receive and adjust an output received from the laser, however, at least a portion of the laser and amplifier share a common waveguide.

In different embodiments of the present invention, properties of the common waveguide such as optical properties, or centerline curvature or cross-sectional are non-uniform along or the waveguide centerline or non-uniform across a normal to the centerline.

Accordingly, a first aspect of the present invention is directed to a widely-tunable diode laser assembly, comprising: an epitaxial structure formed on a substrate, wherein the epitaxial structure has areas of differing optical properties that define active and passive waveguide sections; a laser formed in the epitaxial structure; and an amplifier formed in the epitaxial structure, at least a portion of the laser and amplifier sharing a common waveguide in a linear arrangement that connects the active and passive sections of the laser and amplifier, wherein at least a portion of the common waveguide in a passive waveguide section of the amplifier is curved to reduce reflections from an output facet.

A second aspect of the present invention is directed to a method of generating an optical signal, comprising: providing a diode laser assembly including an epitaxial structure formed on a substrate, wherein the epitaxial structure has areas of differing optical properties that define active and passive waveguide sections, a laser and an amplifier formed in the epitaxial structure, at least a portion of the laser and amplifier sharing a common waveguide in a linear arrangement that connects the active and passive sections of the laser and amplifier, wherein at least a portion of the common waveguide in a passive waveguide section of the amplifier is curved to reduce reflections from an output facet; producing a widely-tunable laser output from the laser, coupling the laser output into the amplifier along the common waveguide; and generating an optical signal from the amplifier.

### BRIEF DESCRIPTION OF THE FIGURES

Figure 1A is a block diagram of a laser assembly that illustrates different functional elements of a laser assembly.
Figure 1B is a cross-sectional view of one embodiment of a widely tunable laser assembly of the present invention and the integration of materials with differing optical properties by an offset quantum well technique.
Figure 2A is a cross sectional view one embodiment of an amplifier illustrating several layer structures and the integration of two materials with differing optical properties by a selected area growth technique..
Figure 2B is a cross sectional view of the Figure 2 assembly illustrating one embodiment for the integration of materials with differing optical properties by a disordered well technique.
Figure 2C is a cross sectional view one embodiment of an amplifier illustrating one embodiment for the integration of several different band gap materials by a butt joint regrowth technique.
Figure 3A is a cross-sectional view of one embodiment of the Figure 1 optical amplifier element where a portion of the waveguide is curved and an interface between an active and a passive section is oblique.
Figure 3B is a cross-sectional view of one embodiment of the Figure 1 optical amplifier element where the amplifier includes a plurality of gain sections.
Figure 3C is a cross-sectional view of one embodiment of the Figure 1 optical amplifier element where the amplifier includes a flared waveguide.
Figure 3D is a cross-sectional view of one embodiment of the Figure 1 optical amplifier element where the amplifier includes a waveguide mode adapter.

### DETAILED DESCRIPTION

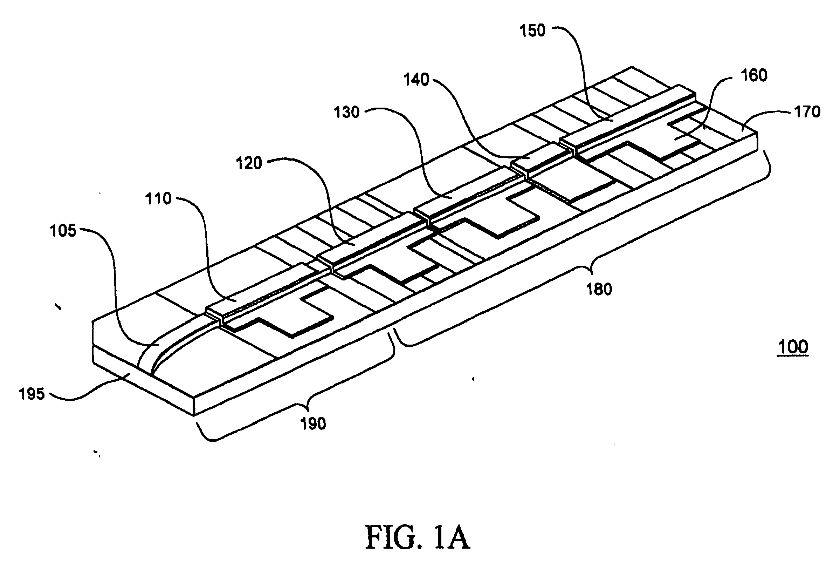

Figure 1A shows a schematic of an embodiment of the invention. In Figure 1A, laser assembly 100, waveguide 105, amplifier gain section 110, front resonator mirror 120, laser gain section 130, laser phase control section 140, back mirror 150 and electrical contact 160, epitaxial structure 170, laser 180, optical amplifier 190 and output facet 195 are shown.

In Figure 1A, laser assembly 100 comprises an integration of a laser and an optical amplifier, with the optical ampliner located external to the laser cavity. Front resonator mirror 120, laser gain section 130, laser phase control section 140, and back mirror 150 form a SGDBR-type laser 180 in epitaxial structure 170. The front and back mirrors define a laser cavity. Amplifier gain section 110 and a portion of waveguide 105 define optical amplifier 190.

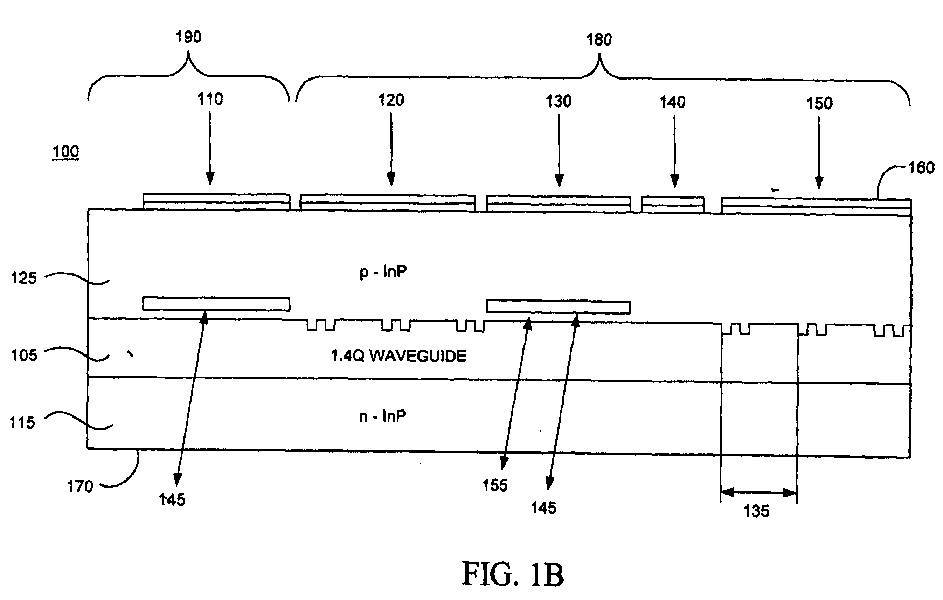

As shown in Figure 1A, despite being external to the laser cavity, the optical amplifier 190 shares a common epitaxial structure 170 with the laser. Epitaxial structure 170 is formed on a substrate (not shown) by processes well-known in the art of semiconductor fabrication. By tailoring optical properties (such as band gap) of different portions of the epitaxial structure, both optically active and optically passive sections can be fabricated in a common structure. Examples of optically active sections of the embodiment shown in Figure 1 are gain sections 110 and 130, phase control section 140 and mirrors 120 and 150. An example of an optically passive section is the portion of waveguide 105 proximal to output facet 195.

According to the invention, at least a portion of laser 180 and optical amplifier 190 share a common waveguide 105. Different portions of the common waveguide 105 may extend through optically active or passive regions. A common waveguide 105 for the laser 180 and optical amplifier 190 enables the output from the laser 180 to be directly coupled into the amplifier 190.

In the embodiment of Figure 1A, amplifier 190 is external to the resonant cavity of laser 180 formed by mirrors 120 and 150. Moreover, amplifier gain section 110 is separately controllable from the laser 180 and is adjustable to increase or decrease the light intensity and output power. The SGBDR laser elements may be controlled separately from the amplifier 190 to tune the laser frequency and otherwise control the input to the optical amplifier 190. By this arrangement of elements, power amplification and tuning functions are substantially uncoupled.

As a result, the optical signal is generated while controlling an intensity of the laser 180 output and maintaining a constant laser 180 wavelength. Moreover, the optical signal generated by the diode laser assembly 100 is tunable over a range of at least 15 nm while maintaining a substantially constant output power. Further, the optical signal may be generated while alternating a propagation direction of the laser 180 output within the amplifier 190, the optical signal may be generated while altering at least one optical mode in the amplifier 190, and the optical signal may be generated while selectively exciting waveguide modes in the amplifier 190.

In the embodiment of Figure 1A, optical amplifier 190 has an active section and a passive section. The active section, amplifier gain section 110, is substantially straight. The passive section of waveguide 105 is curved and intersects output facet 195 at an oblique angle. Both waveguide 105 curvature and the oblique intersection with the output facet act to prevent reflections at the output facet 195 from coupling back into the optical amplifier 190 and laser 180.

Figure 1B shows a longitudinal cross section of a laser assembly 100 of Figure 1A. In Figure 1B, laser assembly 100, waveguide 105, amplifier gain section 110, front resonator mirror 120, laser gain section 130, laser phase control section 140, back mirror 150 and electrical contact 160, epitaxial structure 170, laser 180, optical amplifier 190, output facet 195, p-type semiconductor layer 125, n-type semiconductor layer 115, mirror sampling period 135, offset quantum wells 145 and stop etch layer 155 are shown.

In Figure 1B, waveguide 105 is formed between p-type and n-type semiconductor layers 125 and 115, respectively. Mirrors 120 and 150 are formed by sample gratings etched in waveguide 105 with sampling period 135, as is well-understood in the art.

Figure 1B illustrates the structure resulting from an offset quantum well technique for optically active and passive section formation. According to the offset quantum well technique, the optically active sections have multiple quantum well layers 145 grown in a region offset from waveguide 105. The multiple quantum well layers are separated from the waveguide by a thin stop etch layer 155. Removal of quantum wells, by etching for example, forms optically passive sections.

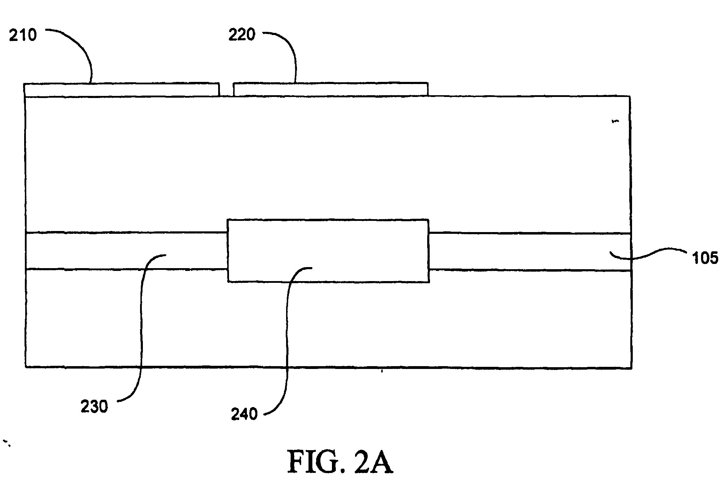

Figures 2A-2C illustrate cross-sectional structures over a portion of laser assembly 100 (see Figure 1) resulting from different techniques for forming optically active and passive sections and their junctions. Figure 2A illustrates a cross-sectional structure over a portion of laser assembly 100 (see Figure 1) resulting from a selected area regrowth technique. The selected area regrowth technique uses a dielectric mask to selectively control the growth rate and composition over different areas of the epitaxial structure. Thus, the material's bandgap can be shifted in certain sections making the material in that section passive or non-absorbing at desired wavelengths. In Figure 2A, optically passive section 210, optically active section 220, bandgap-shifted quantum wells 230, active section quantum wells 240, and waveguide 105 (see Figures 1A-1B) are shown. In Figure 2A, different portions of waveguide 105 are optically active or passive due to bandgap shifting of the quantum wells within the waveguide 105.

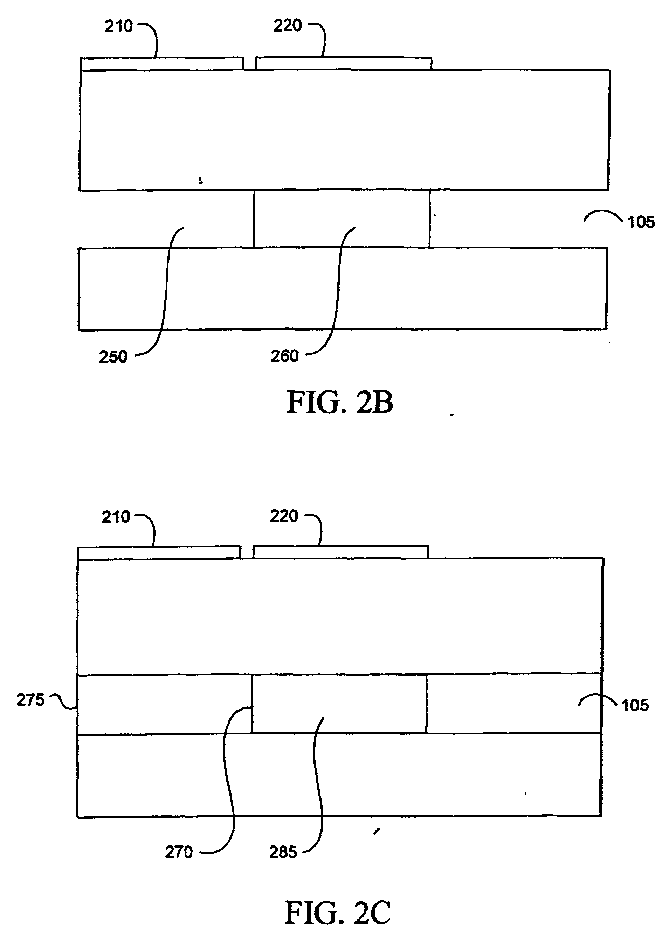

Figure 2B illustrates a cross-sectional structure over a portion of laser assembly 100 (see Figure 1) resulting from a selected area disordering technique for forming optically active and passive sections. The selected area disordering technique uses a dielectric cap or ion implantation to introduce vacancies which can be diffused through an active region to disorder the quantum wells by intermixing them. This disordering shifts quantum well bandgaps, creating optically passive waveguide sections.

In Figure 2B, optically passive section 210, optically active section 220, disordered wells 250, active section multiple quantum wells 260, and waveguide 105 (see Figures 1A-1B) are shown. In Figure 2B, different portions of waveguide 105, sections 210 and 220, are optically active or passive due to the organization of the quantum wells within the waveguide material.

Figure 2C illustrates a cross-sectional structure over a portion of laser assembly 100 (see Figure 1) resulting from a butt joint regrowth technique for forming optically active and passive sections. According to the butt joint regrowth technique, the entire waveguide is etched away in optically passive sections and an optically passive waveguide is grown again. The newly grown portion of the waveguide is butted up against the active waveguide. In Figure 2B, optically passive section 210, optically active section 220, active, butt-joint interface 270, passive waveguide section 275, active waveguide section 285 and waveguide 105 (see Figures 1A-1B) are shown. In Figure 2B, active waveguide section 285 and passive waveguide section 275 are separated by a distinct large gradient butt-joint interface 270 as a result of the etch removal process.

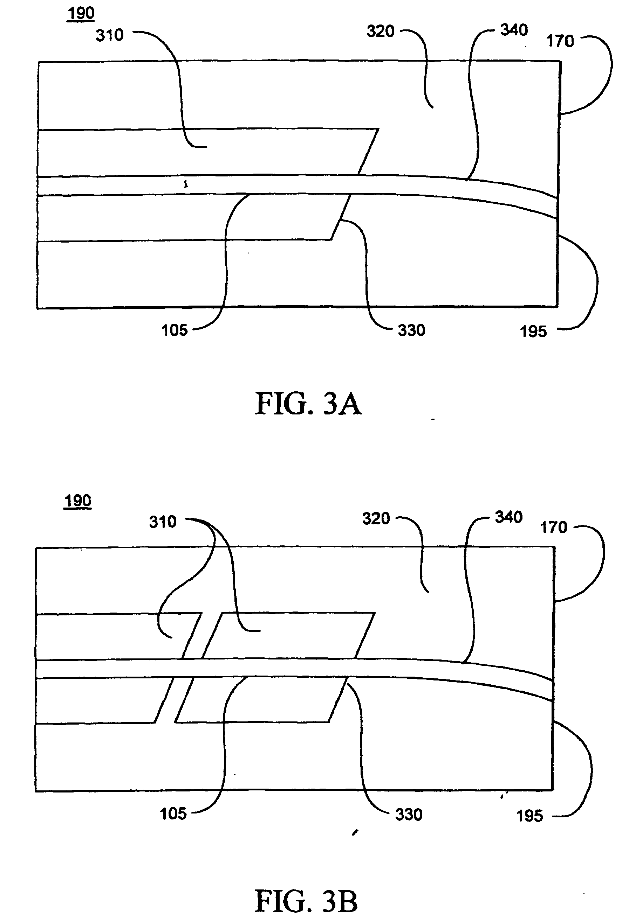

Figures 3A-3D are plan views, illustrating different embodiments of optical amplifier 190 (see Figure 1). In Figures 3A-3D optical amplifier 190, waveguide 105, epitaxial structure 170, output facet 195, active amplifier section 310, passive amplifier section 320, active-passive junction 330, curved waveguide portion 340, flared waveguide portions 350 and 355 and waveguide mode adapter 360 are shown.

In Figure 3A, optical amplifier 190 has an active amplifier section 310 combined with a passive amplifier section 320, where the passive amplifier section includes curved waveguide portion 340. The curved waveguide portion 340 intersects output facet 195 at an oblique angle. Both the waveguide 105 curvature and oblique intersection significantly reduces the amount of light reflecting from the output facet back into the amplifier 190 and laser. Active-passive junction 330 is preferably oblique to a centerline of waveguide 105 so that any reflections from this interface coupling back into the amplifier 190 and laser 180 will be reduced. However, alternate embodiments may have active-passive junction 330 substantially normal to a centerline of the waveguide 105.

Figure 3B shows an alternate embodiment where the amplifier active section has been segmented into a plurality of active sections in order to increase the amplifier output power and reduce a noise figure. In this embodiment shown in Figure 3B, the amplifier active section is segmented into two amplifier active sections 310 that may be independently controllable. Other embodiments have more than two amplifier active sections. This segmenting of the amplifier enables the use of different bias points for the different sections. Having a plurality of amplifier stages allows higher saturated output powers to be reached with better noise performance.

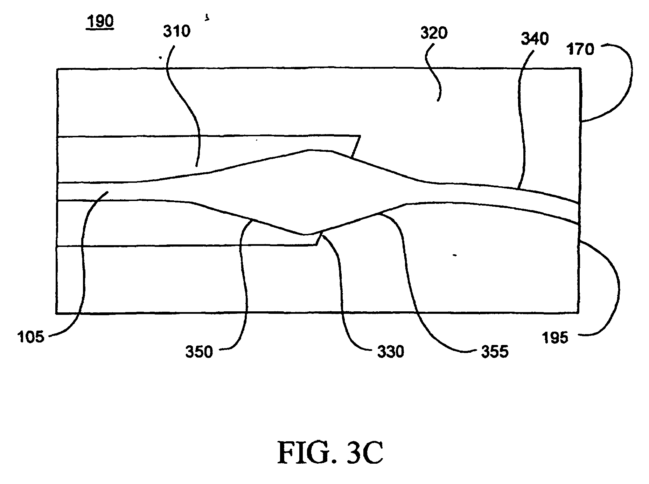

Figure 3C shows an alternate embodiment where a waveguide portion in the amplifier active section is flared, or tapered, to increase the saturated output power. Flared waveguide portion 350 increases the amplifier active volume as compared to the embodiment shown in Figure 3A and decreases the photon density. To accomplish this effectively without introducing significant fiber coupling difficulties it is preferable to use an adiabatic flare, wherein there is no energy transfer across optical modes over the flare to a wider waveguide cross-section. In a preferred embodiment, a second flared-down section 355 to a narrow waveguide cross-section is positioned in the amplifier optically passive section 320 since it is difficult to couple effectively from a wide waveguide into a single mode fiber at output facet 195. In a preferred embodiment, such a flared-down portion is before a curved waveguide portion 340, otherwise, higher order modes will be excited when curving the wide waveguide. In the embodiment shown in Figure 3C, active-passive junction 330 is angled so that any reflections from this interface coupling back into the amplifier and laser will be reduced.

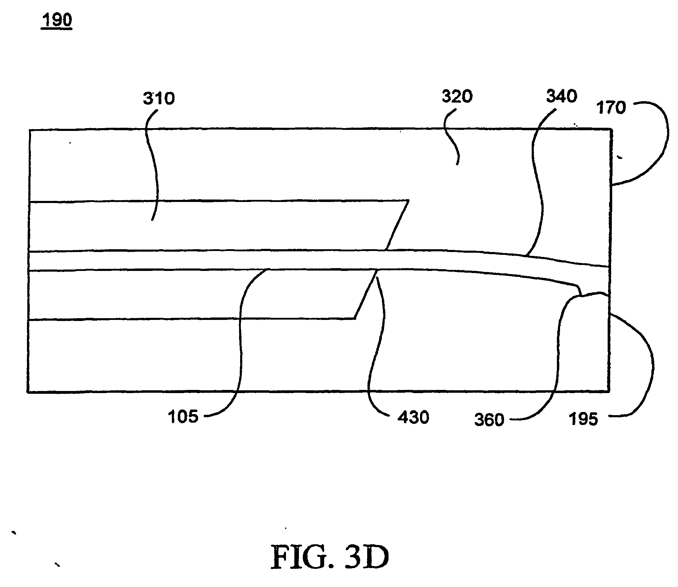

Figure 3D shows another embodiment including a waveguide mode adapter 360. A waveguide mode adapter 360 is preferred in many embodiments to enlarge the optical mode near output facet 195 so that it is more closely matched to the mode in an optical fiber that, as an element in a communications system, may carry the light away from the output facet. Including a waveguide mode adapter 360 thus reduces the fiber coupling loss and increases the alignment tolerances between laser assembly 100 (see Figure 1) and an optical fiber of another system. An embodiment of a waveguide mode adapter 360 includes a section of passive waveguide 105 wherein the waveguide's cross sectional is varied to alter the optical mode in an adiabatic manner.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims.

## Claims

1. A widely-tunable diode laser assembly (100), comprising:
an epitaxial structure (170) formed on a substrate, wherein the epitaxial structure (170) has areas of differing optical properties that define active and passive waveguide sections;
a laser (180) formed in the epitaxial structure (170); and
an amplifier (190) formed in the epitaxial structure (170), at least a portion of the laser (180) and amplifier (190) sharing a common waveguide (105) in a linear arrangement that connects the active and passive sections of the laser (180) and amplifier (190), wherein at least a portion of the common waveguide (105) in a passive waveguide section of the amplifier (190) is curved to reduce reflections from an output facet (195).

2. The diode laser assembly (100) of claim 1, wherein the common waveguide (105) has non-uniform optical properties along its centerline.

3. The diode laser assembly (100) of claim 1, wherein the common waveguide (105) has non-uniform cross-sectional area along its centerline.

4. The diode laser assembly (100) of claim 1, wherein the common waveguide (105) has non-uniform curvature along its centerline.

5. The diode laser assembly (100) of claim 1, wherein the common waveguide (105) has non-uniform optical properties normal to its centerline.

6. The diode laser assembly (100) of claim 1, wherein the amplifier (190) includes at least one active region (310) and at least one passive region (320).

7. The diode laser assembly (100) of claim 6, wherein the waveguide (105) extends through an active region (310) and a passive region (320).

8. The diode laser assembly (100) of claim 7, wherein the amplifier (190) includes a flared waveguide section (350,355).

9. The diode laser assembly (100) of claim 7, wherein an interface (330) between the active region (310) and the passive region (320) is oblique to a centerline of the waveguide (105).

10. The diode laser assembly (100) of claim 7, wherein an interface (330) between the active region (310) and the passive region (320) is substantially normal to a centerline of the waveguide (105).

11. The diode laser assembly (100) of claim 7, wherein an end of the waveguide (105) in the amplifier (190) terminates at an oblique angle to the output facet (195).

12. The diode laser assembly (100) of claim 6, wherein the waveguide (105) includes a waveguide mode adapter (360).

13. The diode laser assembly (100) of claim 1, wherein at least a portion (350,355) of the waveguide (105) is flared.

14. The diode laser assembly (100) of claim 21, wherein a flared portion (350) of the waveguide (105) is in an active region (310).

15. The diode laser assembly (100) of claim 21, wherein a flared portion (355) of the waveguide (105) is in a passive region (320).

16. The diode laser assembly (100) of claim 1, wherein the waveguide (105) includes an active section.

17. The diode laser assembly (100) of claim 16, wherein the active section of the waveguide (105) is positioned in the first active section (310) of the amplifier (190).

18. The diode laser assembly (100) of claim 16, wherein the active section of the waveguide (105) is positioned in the second active section (310) of the amplifier (190).

19. The diode laser assembly (100) of claim 6, wherein the first active region (310) has an oblique distal face.

20. The diode laser assembly (100) of claim 6, wherein the amplifier (190) includes a plurality of independently controllable active regions (310).

21. The diode laser assembly (100) of claim 20, wherein a first and a second active region (310) are separated by a passive region (320).

22. The diode laser assembly (100) of claim 21, wherein the first active region (310) has an oblique distal face.

23. The diode laser assembly (100) of claim 21, wherein the second active region (310) has an oblique proximal face.

24. The diode laser assembly (100) of claim 21, wherein the oblique distal face of the first active region (310) is parallel to the oblique proximal face of the second active region (310).

25. The diode laser assembly (100) of claim 21, wherein the second active region (310) has an oblique distal face.

26. The diode laser assembly (100) of claim 25, wherein the proximal face and the distal face of the second region (310) are parallel.

27. The diode laser assembly (100) of claim 1, wherein the laser (180) includes first and second reflectors (120,150) and at least one of the first and second reflectors (120,150) is tunable.

28. The diode laser assembly (100) of claim 27, wherein at least one of the first and second reflectors (120,150) is a distributed reflector.

29. The diode laser assembly (100) of claim 27, wherein both of the first and second reflectors (120,150) are distributed reflectors.

30. The diode laser assembly (100) of claim 27, wherein at least one of the first and second reflectors (120,150) is a distributed Bragg reflector.

31. The diode laser assembly (100) of claim 27, wherein each of the first and second reflectors (120,150) is a distributed Bragg reflector.

32. The diode laser assembly (100) of claim 27, wherein a maximum reflectivity of at least one of the first and second reflectors (120,150) is tunable.

33. The diode laser assembly (100) of claim 27, wherein a maximum reflectivity of each of the first and second reflectors (120,150) is tunable.

34. The diode laser assembly (100) of claim 27, wherein the maximum reflectivities of each of the first and second reflectors (120,150) are tunable relative to each other.

35. The diode laser assembly (100) of claim 1, wherein the laser (180) has a multi-active region gain medium.

36. The diode laser assembly (100) of claim 27, wherein the laser (180) includes a controllable amplifier (190) positioned outside of the laser (180).

37. The diode laser assembly (100) of claim 1, wherein an optical signal generated by the diode laser assembly (100) is tunable within a range of at least 15 nm.

38. The diode laser assembly (100) of claim 1, wherein an optical signal generated by the diode laser assembly (100) is tunable over a tuning range while maintaining a substantially constant output power.

39. The diode laser assembly (100) of claim 1, wherein an optical signal generated by the diode laser assembly (100) is tunable over a tuning range of at least 15 nm while maintaining a substantially constant output power.

40. A method of generating an optical signal, comprising:
providing a diode laser assembly (100) including an epitaxial structure (170) formed on a substrate, wherein the epitaxial structure (170) has areas of differing optical properties that define active and passive waveguide sections, a laser (180) and an amplifier (190) formed in the epitaxial structure (170), at least a portion of the laser (180) and amplifier (190) sharing a common waveguide (105) in a linear arrangement that connects the active and passive sections of the laser (180) and amplifier (190), wherein at least a portion of the common waveguide (105) in a passive waveguide section of the amplifier (190) is curved to reduce reflections from an output facet (195);
producing a widely-tunable laser output from the laser (180);
coupling the laser output into the amplifier (190) along the common waveguide (105); and
generating an optical signal from the amplifier (190).

41. The method of claim 40, wherein the optical signal is generated while controlling an intensity of the laser output and maintaining a constant laser wavelength.

42. The method of claim 40, wherein the optical signal is tunable within a range of at least 15 nm.

43. The method of claim 40, wherein the optical signal is tunable over a tuning range while maintaining a substantially constant output power.

44. The method of claim 40, wherein the optical signal is tunable over a tuning range of at least 15 nm while maintaining a substantially constant output power.

45. The method of claim 40, wherein the optical signal is generated while alternating a propagation direction of the laser output within the amplifier (190).

46. The method of claim 40, wherein the optical signal is generated while minimizing back reflections into the laser (180).

47. The method of claim 40, wherein the optical signal is generated while altering at least one optical mode in the amplifier (190).

48. The method of claim 40, further comprising altering at least one optical mode in an adiabatic manner.

49. The method of claim 40, wherein the optical signal is generated while selectively exciting waveguide modes in the amplifier (190).

## Patentansprüche

1. Breit abstimmbare Diodenlaseranordnung (100) mit:
einer auf einem Substrat gebildeten epitaktischen Struktur (170), wobei die epitaktische Struktur (170) Bereiche mit sich unterscheidenden optischen Eigenschaften aufweist, welche aktive und passive Wellenleiterabschnitte definieren,
einem in der epitaktischen Struktur (170) gebildeten Laser (180) und
einem in der epitaktischen Struktur (170) gebildeten Verstärker (190), wobei mindestens ein Teil des Lasers (180) und des Verstärkers (190) sich einen gemeinsamen Wellenleiter (105) in einer linearen Anordnung teilen, der die aktiven und passiven Bereiche des Lasers (180) und des Verstärkers (190) verbindet, wobei mindestens ein Teil des gemeinsamen Wellenleiters (105) in einem passiven Wellenleiterabschnitt des Verstärkers (190) gekrümmt ist, so daß Reflexionen von einer Ausgangsfacette (195) reduziert werden.

2. Diodenlaseranordnung (100) gemäß Anspruch 1, **dadurch gekennzeichnet, daß** der gemeinsame Wellenleiter (105) ungleichmäßige optische Eigenschaften entlang seiner Mittelachse aufweist.

3. Diodenlaseranordnung (100) nach Anspruch 1, **dadurch gekennzeichnet, daß** der gemeinsame Wellenleiter (105) eine ungleichmäßige Querschnittsfläche entlang seiner Mittelachse aufweist.

4. Diodenlaseranordnung (100) nach Anspruch 1, **dadurch gekennzeichnet, daß** der gemeinsame Wellenleiter (105) eine ungleichmäßige Krümmung entlang seiner Mittelachse aufweist.

5. Diodenlaseranordnung (100) nach Anspruch 1, **dadurch gekennzeichnet, daß** der gemeinsame Wellenleiter (105) ungleichmäßige optische Eigenschaften senkrecht zu seiner Mittelachse aufweist.

6. Diodenlaseranordnung (100) nach Anspruch 1, **dadurch gekennzeichnet, daß** der Verstärker (190) mindestens einen aktiven Bereich (310) und mindestens einen passiven Bereich (320) aufweist.

7. Diodenlaseranordnung (100) nach Anspruch 6, **dadurch gekennzeichnet, daß** der Wellenleiter (105) sich über einen aktiven Bereich (310) und über einen passiven Bereich (320) erstreckt.

8. Diodenlaseranordnung (100) nach Anspruch 7, **dadurch gekennzeichnet, daß** der Verstärker (190) einen trichterförmigen Wellenleiterabschnitt (350, 355) aufweist.

9. Diodenlaseranordnung (100) nach Anspruch 7, **dadurch gekennzeichnet, daß** eine Grenzfläche (330) zwischen dem aktiven Bereich (310) und dem passiven Bereich (320) schräg zu einer Mittelachse des Wellenleiters (105) ist.

10. Diodenlaseranordnung (100) nach Anspruch 7, **dadurch gekennzeichnet, daß** eine Grenzfläche (330) zwischen dem aktiven Bereich (310) und dem passiven Bereich (320) im wesentlichen senkrecht zu einer Mittelachse des Wellenleiters (105) ist.

11. Diodenlaseranordnung (100) nach Anspruch 7, **dadurch gekennzeichnet, daß** ein Ende des Wellenleiters (105) in dem Verstärker (190) unter einem schiefen Winkel zu der Ausgangsfacette (195) endet.

12. Diodenlaseranordnung (100) nach Anspruch 6, **dadurch gekennzeichnet, daß** der Wellenleiter (105) einen Wetienteitermodenadapter (360) aufweist.

13. Diodenlaseranordnung (100) nach Anspruch 1, **dadurch gekennzeichnet, daß** mindestens ein Teil (350, 355) des Wellenleiters (105) trichterförmig ist.

14. Diodenlaseranordnung (100) nach Anspruch 21, **dadurch gekennzeichnet, daß** ein trichterförmiger Teil (350) des Wellenleiters (105) in einem aktiven Bereich (310) liegt.

15. Diodenlaseranordnung (100) nach Anspruch 21, **dadurch gekennzeichnet, daß** ein trichterförmiger Bereich (355) des Wellenleiters (105) in einem passiven Bereich (320) liegt.

16. Diodenlaseranordnung (100) nach Anspruch 1, **dadurch gekennzeichnet, daß** der Wellenleiter (105) einen aktiven Abschnitt aufweist.

17. Diodenlaseranordnung (100) nach Anspruch 16, **dadurch gekennzeichnet, daß** der aktive Abschnitt des Wellenleiters (105) in dem ersten aktiven Abschnitt (310) des Verstärkers (190) angeordnet ist.

18. Diodenlaseranordnung (100) nach Anspruch 16, **dadurch gekennzeichnet, daß** der aktive Abschnitt des Wellenleiters (105) in dem zweiten aktiven Abschnitt (310) des Verstärkers (190) angeordnet ist.

19. Diodenlaseranordnung (100) gemach Anspruch 6, **dadurch gekennzeichnet, daß** der erste aktive Bereich (310) eine schräge distale Fläche aufweist.

20. Diodenlaseranordnung (100) nach Anspruch 6, **dadurch gekennzeichnet, daß** der Verstärker (190) eine Mehrzahl von unabhängig steuerbaren aktiven Bereichen (310) aufweist.

21. Diodenlaseranordnung (100) nach Anspruch 20, **dadurch gekennzeichnet, daß** ein erster und ein zweiter aktiver Bereich (310) durch einen passiven Bereich (320) getrennt sind.

22. Diodenlaseranordnung (100) nach Anspruch 21, **dadurch gekennzeichnet, daß** der erste aktive Bereich (310) eine schräge distale Fläche aufweist.

23. Diodenlaseranordnung (100) nach Anspruch 21, **dadurch gekennzeichnet, daß** der zweite aktive Bereich (310) eine schräge proximale Fläche aufweist.

24. Diodenlaseranordnung (100) nach Anspruch 21, **dadurch gekennzeichnet, daß** die schräge distale Fläche des ersten aktiven Bereichs (310) parallel zu der schrägen proximalen Fläche des zweiten aktiven Bereichs (310) ist.

25. Diodenlaseranordnung (100) nach Anspruch 21, **dadurch gekennzeichnet, daß** der zweite aktive Bereich (310) eine schräge distale Fläche aufweist.

26. Diodenlaseranordnung (100) nach Anspruch 25, **dadurch gekennzeichnet, daß** die proximale Fläche und die distale Fläche des zweiten Bereichs (310) parallel sind.

27. Diodenlaseranordnung (100) nach Anspruch 1, **dadurch gekennzeichnet, daß** der Laser (180) erste und zweite Reflektoren (120, 150) aufweist und mindestens einer der ersten und zweiten Reflektoren (120, 150) abstimmbar ist.

28. Diodenlaseranordnung (100) nach Anspruch 27, **dadurch gekennzeichnet, daß** mindestens einer der ersten und zweiten Reflektoren (120, 150) ein verteilter Reflektor ist.

29. Diodenlaseranordnung (100) nach Anspruch 27, **dadurch gekennzeichnet, daß** sowohl der erste als auch der zweite Reflektor (120, 150) verteilte Reflektoren sind.

30. Diodenlaseranordnung (100) nach Anspruch 27, **dadurch gekennzeichnet, daß** mindestens einer der ersten und zweiten Reflektoren (120, 150) ein verteilter Bragg-Reflektor ist.

31. Diodenlaseranordnung (100) nach Anspruch 27, **dadurch gekennzeichnet, daß** sowohl der erste als auch der zweite Reflektor (120, 150) ein verteilter Bragg-Reflektor ist.

32. Diodenlaseranordnung (100) nach Anspruch 27, **dadurch gekennzeichnet, daß** eine maximale Reflektivität mindestens eines der ersten und zweiten Reflektoren (120, 150) abstimmbar ist.

33. Diodenlaseranordnung (100) nach Anspruch 27, **dadurch gekennzeichnet, daß** eine maximale Reflektivität sowohl des ersten als auch des zweiten Reflektors (120, 150) abstimmbar ist.

34. Diodenlaseranordnung (100) nach Anspruch 27, **dadurch gekennzeichnet, daß** die maximale Reflektivitäten jede der ersten und zweiten Reflektoren (102, 150) relativ zueinander abstimmbar sind.

35. Diodenlaseranordnung (100) nach Anspruch 1, **dadurch gekennzeichnet, daß** der Laser (180) ein Verstärkungsmedium mit mehrfach aktivem Bereich aufweist.

36. Diodenlaseranordnung (100) nach Anspruch 27, **dadurch gekennzeichnet, daß** der Laser (180) einen steuerbaren Verstärker (190) aufweist, der außerhalb des Lasers (180) angeordnet ist.

37. Diodenlaseranordnung (100) nach Anspruch 1, **dadurch gekennzeichnet, daß** ein optisches Signal, das durch die Diodenlaseranordnung (100) erzeugt wird, innerhalb eines Bereichs von mindestens 15 nm abstimmbar ist.

38. Diodenlaseranordnung (100) nach Anspruch 1, **dadurch gekennzeichnet, daß** ein optisches Signal, das von der Diodenlaseranordnung (100) erzeugt wird, über einen Abstimmbereich abstimmbar ist, während eine im wesentlichen konstante Ausgangsleistung erhalten bleibt.

39. Diodenlaseranordnung (100) nach Anspruch 1, **dadurch gekennzeichnet, daß** ein durch die Diodenlaseranordnung (100) erzeugtes optisches Signal über einen Abstimmbereich von mindestens 15 nm abstimmbar ist, während eine im wesentlichen konstante Ausgangsleistung erhalten bleibt.

40. Verfahren zum Erzeugen eines optischen Signals, mit:
Bereitstellen einer Diodenlaseranordnung (100) mit einer auf einem Substrat gebildeten epitaktischen Struktur (170), wobei die epitaktische Struktur (170) Bereiche mit voneinander verschiedenen optischen Eigenschaften aufweist, die aktive und passive Wellenleiterabschnitte definieren, mit einem Laser (180) und einem Verstärker (190), die in der epitaktischen Struktur (170) gebildet sind, wobei mindestens ein Teil des Lasers (180) und des Verstärkers (190) sich einen gemeinsamen Wellenleiter (105) in einer linearen Anordnung teilen, der die aktiven und passiven Abschnitte des Lasers (180) und Verstärkers (190) verbindet, wobei mindestens ein Teil des gemeinsamen Wellenleiters (105) in einem passiven Wellenleiterabschnitt des Verstärkers (190) gekrümmt ist, so daß Reflexionen von einer Ausgangsfacette (195) reduziert werden,
Erzeugen eines breit abstimmbaren Laserausgangs des Lasers (180),
Einkoppeln des Laserausgangs in den Verstärker (190) entlang des gemeinsamen Wellenleiters (105) und
Erzeugen eines optischen Signals aus dem Verstärker (190).

41. Verfahren nach Anspruch 40, **dadurch gekennzeichnet, daß** das optische Signal erzeugt wird während eine Intensität des Laserausgangs gesteuert wird und eine konstante Laserwellenlänge erhalten wird.

42. Verfahren nach Anspruch 40, **dadurch gekennzeichnet, daß** das optische Signal innerhalb eines Bereichs von mindestens 15 nm abstimmbar ist.

43. Verfahren nach Anspruch 40, **dadurch gekennzeichnet, daß** das optische Signal über einen Abstimmbereich abstimmbar ist, während eine im wesentlichen konstante Ausgangsleistung erhalten wird.

44. Verfahren nach Anspruch 40, **dadurch gekennzeichnet, daß** das optische Signal über einen Abstimmbereich von mindestens 15 nm abstimmbar ist, während eine im wesentlichen konstante Ausgangsleistung erhalten wird.

45. Verfahren nach Anspruch 40, **dadurch gekennzeichnet, daß** das optische Signal erzeugt wird, während eine Ausbreitungsrichtung des Laserausgangs innerhalb des Verstärkers (190) wechselt.

46. Verfahren nach Anspruch 40, **dadurch gekennzeichnet, daß** das optische Signal erzeugt wird, während Rückreflexionen in den Laser (180) minimiert werden.

47. Verfahren nach Anspruch 40, **dadurch gekennzeichnet, daß** das optische Signal erzeugt wird, während mindestens eine optische Mode in dem Verstärker (190) verändert wird.

48. Verfahren nach Anspruch 40, darüber hinaus mit Ändem mindestens einer optischen Mode auf eine adiabatische Weise.

49. Verfahren nach Anspruch 40, **dadurch gekennzeichnet, daß** das optische Signal erzeugt wird, während selektiv Wellenteitermoden in dem Verstärker (190) angeregt werden.

## Revendications

1. Assemblage de diode laser accordable dans une large bande (100) comprenant :
une structure épitaxiale (170) formée sur un substrat, dans lequel la structure épitaxiale (170) a des zones ayant des propriétés optiques différentes qui définissent des sections de guides d'ondes actives et passives ;
un laser (180) formé dans la structure épitaxiale (170) ; et
un amplificateur (190) formé dans la structure épitaxiale (170), au moins une partie du laser (180) et de l'amplificateur (190) partageant un guide d'ondes commun (105) dans une configuration linéaire qui connecte les sections actives et passives du laser (180) et de l'amplificateur (190), dans lequel au moins une partie du guide d'ondes commun (105) dans une section de guide d'ondes passive de l'amplificateur (190) est incurvée pour réduire les réflexions provenant d'une facette de sortie (195).

2. Assemblage de diode laser (100) selon la revendication 1, dans lequel le guide d'ondes commun (105) a des propriétés optiques non uniformes le long de son axe central.

3. Assemblage de diode laser (100) selon la revendication 1, dans lequel le guide d'ondes commun (105) a une superficie en section transversale non uniforme le long de son axe central.

4. Assemblage de diode laser (100) selon la revendication 1, dans lequel le guide d'ondes commun (105) a une courbure non uniforme le long de son axe central.

5. Assemblage de diode laser (100) selon la revendication 1, dans lequel le guide d'ondes commun (105) a des propriétés optiques non uniformes perpendiculairement à son axe central.

6. Assemblage de diode laser (100) selon la revendication 1, dans lequel l'amplificateur (190) comporte au moins une région active (310) et au moins une région passive (320).

7. Assemblage de diode laser (100) selon la revendication 6, dans lequel le guide d'ondes (105) s'étend à travers une région active (310) et une région passive (320).

8. Assemblage de diode laser (100) selon la revendication 7, dans lequel l'amplificateur (190) comporte une section de guide d'ondes évasée (350, 355).

9. Assemblage de diode laser (100) selon la revendication 7, dans lequel une interface (330) entre la région active (310) et la région passive (320) est oblique par rapport à un axe central du guide d'ondes (105).

10. Assemblage de diode laser (100) selon la revendication 7, dans lequel une interface (330) entre la région active (310) et la région passive (320) est sensiblement perpendiculaire à un axe central du guide d'ondes (105).

11. Assemblage de diode laser (100) selon la revendication 7, dans lequel une extrémité du guide d'ondes (105) dans l'amplificateur (190) se termine par un angle oblique par rapport à la facette de sortie (195).

12. Assemblage de diode laser (100) selon la revendication 6, dans lequel le guide d'ondes (105) comporte un adaptateur de mode de guide d'ondes (360).

13. Assemblage de diode laser (100) selon la revendication 1, dans lequel au moins une partie (350, 355) du guide d'ondes (105) est évasée.

14. Assemblage de diode laser (100) selon la revendication 21, dans lequel une partie évasée (350) du guide d'ondes (105) est dans une région active (310).

15. Assemblage de diode laser (100) selon la revendication 21, dans lequel une partie évasée (355) du guide d'ondes (105) est dans une région passive (320).

16. Assemblage de diode laser (100) selon la revendication 1, dans lequel le guide d'ondes (105) comporte une section active.

17. Assemblage de diode laser (100) selon la revendication 16, dans lequel la section active du guide d'ondes (105) est positionnée dans la première section active (310) de l'amplificateur (190).

18. Assemblage de diode laser (100) selon la revendication 16, dans lequel la section active du guide d'ondes (105) est positionnée dans la seconde section active (310) de l'amplificateur (190).

19. Assemblage de diode laser (100) selon la revendication 6, dans lequel la première région active (310) a une face distante oblique.

20. Assemblage de diode laser (100) selon la revendication 6, dans lequel l'amplificateur (190) comporte une pluralité de régions actives pouvant être commandées indépendamment (310).

21. Assemblage de diode laser (100) selon la revendication 20, dans lequel des première et seconde régions actives (310) sont séparées par une région passive (320).

22. Assemblage de diode laser (100) selon la revendication 21, dans lequel la première région active (310) a une face distante oblique.

23. Assemblage de diode laser (100) selon la revendication 21, dans lequel la seconde région active (310) a une face proche oblique.

24. Assemblage de diode laser (100) selon la revendication 21, dans lequel la face distante oblique de la première région active (310) est parallèle à la face proche oblique de la seconde région active (310).

25. Assemblage de diode laser (100) selon la revendication 21, dans lequel la seconde région active (310) a une face distante oblique.

26. Assemblage de diode laser (100) selon la revendication 25, dans lequel la face proche et la face distante de la seconde région (310) sont parallèles.

27. Assemblage de diode laser (100) selon la revendication 1, dans lequel le laser (180) comporte des premier et second réflecteurs (120, 150) et au moins l'un des premier et second réflecteurs (120, 150) est accordable.

28. Assemblage de diode laser (100) selon la revendication 27, dans lequel au moins l'un des premier et second réflecteurs (120, 150) est un réflecteur réparti.

29. Assemblage de diode laser (100) selon la revendication 27, dans lequel les premier et second réflecteurs (120, 150) sont tous deux des réflecteurs répartis.

30. Assemblage de diode laser (100) selon la revendication 27, dans lequel au moins l'un des premier et second réflecteurs (120, 150) est un réflecteur de Bragg réparti.

31. Assemblage de diode laser (100) selon la revendication 27, dans lequel chacun des premier et second réflecteurs (120, 150) est un réflecteur de Bragg réparti.

32. Assemblage de diode laser (100) selon la revendication 27, dans lequel une réflectivité maximum d'au moins l'un des premier et second réflecteurs (120, 150) est accordable.

33. Assemblage de diode laser (100) selon la revendication 27, dans lequel une réflectivité maximum de chacun des premier et second réflecteurs (120, 150) est accordable.

34. Assemblage de diode laser (100) selon la revendication 27, dans lequel les réflectivités maximum de chacun des premier et second réflecteurs (120, 150) sont accordables l'une par rapport à l'autre.

35. Assemblage de diode laser (100) selon la revendication 1, dans lequel le laser (180) a un milieu à gain à multiples régions actives.

36. Assemblage de diode laser (100) selon la revendication 27, dans lequel le laser (180) comporte un amplificateur pouvant être commandé (190) positionné à l'extérieur du laser (180).

37. Assemblage de diode laser (100) selon la revendication 1, dans lequel un signal optique généré par l'assemblage de diode laser (100) est accordable à l'intérieur d'une plage d'au moins 15 nm.

38. Assemblage de diode laser (100) selon la revendication 1, dans lequel un signal optique généré par l'assemblage de diode laser (100) est accordable dans une plage d'accord tout en maintenant sensiblement constante la puissance de sortie.

39. Assemblage de diode laser (100) selon la revendication 1, dans lequel un signal optique généré par l'assemblage de diode laser (100) est accordable dans une plage d'accord d'au moins 15 nm tout en maintenant sensiblement constante la puissance de sortie.

40. Procédé pour produire un signal optique comprenant :
l'utilisation d'un assemblage de diode laser (100) comportant une structure épitaxiale (170) formée sur un substrat, dans lequel la structure épitaxiale (170) a des zones ayant des propriétés optiques différentes qui définissent des sections de guide d'ondes actives et passives, un laser (180) et un amplificateur (190) formés dans la structure épitaxiale, au moins une partie du laser (180) et l'amplificateur (190) partageant un guide d'ondes commun (105) dans une configuration linéaire qui connecte les sections actives et passives du laser (180) et de l'amplificateur (190), dans lequel au moins une partie du guide d'ondes commun (105) dans une section de guide d'ondes passive de l'amplificateur (190) est incurvée pour réduire les réflexions provenant d'une facette de sortie (195) ;
produire une sortie laser accordable dans une large gamme à partir du laser (180) ;
appliquer la sortie du laser à l'amplificateur (190) le long du guide d'ondes commun (105) ; et
générer un signal optique à partir de l'amplificateur (190).

41. Procédé selon la revendication 40, dans lequel le signal optique est généré tout en commandant une intensité de la sortie laser et en maintenant une longueur d'onde laser constante.

42. Procédé selon la revendication 40, dans lequel le signal optique est accordable à l'intérieur d'une plage d'au moins 15 nm.

43. Procédé selon la revendication 40, dans lequel le signal optique est accordable sur une plage d'accord tout en maintenant une puissance de sortie sensiblement constante.

44. Procédé selon la revendication 40, dans lequel le signal optique est accordable dans une plage d'accord d'au moins 15 nm tout en maintenant une puissance de sortie sensiblement constante.

45. Procédé selon la revendication 40, dans lequel le signal optique est généré tout en alternant la direction de propagation de la sortie du laser à l'intérieur de l'amplificateur (190).

46. Procédé selon la revendication 40, dans lequel le signal optique est généré tout en minimisant les rétro-réflexions à l'intérieur du laser (180).

47. Procédé selon la revendication 40, dans lequel le signal optique est généré tout en modifiant au moins un mode optique dans l'amplificateur (190).

48. Procédé selon la revendication 40, comprenant en outre la modification adiabatique d'au moins un mode optique.

49. Procédé selon la revendication 40, dans lequel le signal optique est généré tout en excitant sélectivement des modes de guide d'ondes dans l'amplificateur (190).
